# EUROPÄISCHE PATENTSCHRIFT

(11) **EP 2 081 661 B1**
(45) Veröffentlichungstag und Bekanntmachung des Hinweises auf die Patenterteilung: **28.03.2018**
(21) Anmeldenummer: 07819132.7
(22) Anmeldetag: 19.10.2007
(51) Int. Cl.: B01D 19/00, C08G 64/40, C08G 65/46

(54) **VERFAHREN ZUR ABTRENNUNG EINER ORGANISCHEN VON EINER ELEKTROLYTHALTIGEN WÄSSRIGEN UND ORGANISCHEN PHASE**
METHOD FOR THE SEPARATION OF AN ORGANIC PHASE FROM AN ELECTROLYTE-CONTAINING AQUEOUS AND ORGANIC PHASE
PROCÉDÉ POUR SÉPARER UNE PHASE ORGANIQUE D'UNE PHASE ORGANIQUE AQUEUSE CONTENANT DES ÉLECTROLYTES

(30) Priorität: 25.10.2006 DE 102006050381
(43) Veröffentlichungstag der Anmeldung: 29.07.2009
(73) Patentinhaber: Covestro Deutschland AG, 51373 Leverkusen (DE)
(72) Erfinder: MEYER, Helmut, 51519 Odenthal (DE); NABERFELD, Guido, 15205-9741 Pittsburgh (US); RECHNER, Johann, 47906 Kempen (DE); WARSITZ, Rafael, 45136 Essen (DE); TRAVING, Michael, 51399 Burscheid (DE); BÄCKER, Werner, 51688 Wipperfürth (DE); ELSNER, Thomas, 40595 Düsseldorf (DE); NENNEMANN, Arno, 51469 Bergisch-Gladbach (DE); BAHNMÜLLER, Stefan, Singapur 049514 (SG); LANGSTEIN, Gerhard, 51515 Kürten (DE); THEATO, Patrick, 55128 Mainz (DE); DUFF, Daniel-Gordon, 51373 Leverkusen (DE); HITZBLECK, Julia, 50668 Köln (DE); KESSLER, Daniel, 56191 Weitersburg (DE); PLUG, Sascha, 51375 Leverkusen (DE)
(74) Vertreter: Levpat
(86) Internationale Anmeldenummer: PCT/EP2007/009067
(87) Internationale Veröffentlichungsnummer: WO 2008/049548

(56) Entgegenhaltungen:
- EP-A- 0 014 462
- WO-A-00/09582
- CA-A- 747 994
- DE-A1- 4 343 754
- DE-A1- 19 510 061
- JP-A- 52 096 697
- JP-A- 52 098 090
- US-A- 5 051 182

## Beschreibung

Die Erfindung betrifft ein neuartiges Verfahren zur Abtrennung einer von Elektrolyt befreiten, organischen Phase aus einem Gemisch von elektrolythaltiger (salzhaltiger) wässriger und organischer Phase. Das elektrolythaltige Wasser liegt dabei dispergiert bzw. emulgiert in der organischen Phase vor.

Die Abtrennung von Elektrolyten (Salze, Laugen, Säuren) und Wasser aus organischen Phasen, wie z. B. mit Wasser nicht mischbare Polymere, Aromate, Aliphate, spielt eine bedeutende Rolle in großtechnischen Prozessen. Nach dem Stand der Technik erfolgt eine derartige Abtrennung mittels Zentrifugation, Abscheidung, Destillation, Sprühtrocknung, Eindampfextrusion, Ausfällung und/oder Faserkoaleszenz. In der Regel erfolgt die Abtrennung der Elektrolyten (Lauge und Säure) erst nach einer Neutralisation zu den entsprechenden Salzen, mit Säuren und Laugen.

Nachteil ist der Verbrauch an Neutralisationsmittel und der damit verbundene Verlust an Lauge und/oder Säure, sowie die Entsorgung/Verwendung des Salzes. Weitere Nachteile der oben beschriebenen Verfahren zur Abtrennung einer elektrolytfreien (Lauge, Säure, Salz) organischen Phase sind, dass sie zum einem einen hohen Einsatz von Energie zur Abtrennung des Wassers und zum anderen eine aufwendige Abtrennung von Restgehalten an Elektrolyten (Salzen) über Waschprozesse erforderlich machen. Hierdurch ergeben sich hohe Kosten wegen der damit verbundenen Wasseraufbereitung und des hohen Energiebedarfs. Tests zur Verbesserung der Phasenseparation, wie z. B. Temperaturwechsel, verbesserte Zentrifugen bzw. Verkleinern der Abscheider unter Einsatz von Koaleszierhilfen, zeigten oftmals eine unzureichende Separation bspw. eine starke Neigung zur Verblockung der Koaleszierhilfen durch Belegung mit Salz.

In dem konkreten Fall des Polyetherpolyol-Herstellungsprozesses erfolgt die Abtrennung von Salzen und Restwassergehalten aus der Polyolphase nach dem Stand der Technik durch Zentrifugation, Abscheidung bzw. Destillation und Filtration (s. z. B. EP 0 038 986 A2).

Großtechnisch werden Polyetherpolyole meist durch Anlagerung von Alkylenoxiden, insbesondere Propylenoxid und/oder Ethylenoxid, an Startverbindungen mit aciden Wasserstoffatomen (z. B. Wasser, Polyalkohole oder Polyamine) in Gegenwart von basischen Substanzen, in der Regel Alkalihydroxiden, wie z. B. KOH oder NaOH, als Katalysator hergestellt. Bei einem der derzeit üblichen Aufarbeitungsverfahren wird der basische Katalysator (z. B. KOH) aus dem alkalischen Polymerisat in mehreren Verfahrensschritten entfernt. Dabei wird zunächst das alkalische Polymerisat, z. B. mit verdünnter Schwefelsäure neutralisiert, wonach die Hauptmenge Wasser, unter gleichzeitiger Kristallisation der anorganischen Salze (hier K₂SO₄) abdestilliert wird. Das ausgefallene Salz wird abfiltriert, worauf das restliche Wasser abdestilliert und die Restmenge an Salz durch Filtration entfernt wird.

Die Nachteile dieser bekannten Neutralisationsverfahren sind zum einen der Verbrauch an Neutralisationssäure und ein sehr hoher Energieverbrauch beim Destillieren des Wassers. Zum anderen ist es aufwendig, das meist sehr feinteilige Salz abzufiltrieren.

Die nach der Polymerisation erhaltene Polyetherpolyolmischung besteht aus einer organischen Phase, die Polyetherpolyol und die bei der Reaktion auftretenden Nebenprodukte (u.a. 1,4-Dioxan, 2,5-Dimethyl-1,4-Dioxan, 2,4-Dimethyl-1,3-Dioxolan, 2-Ethyl-4-Methyl-1,3-Dioxolan, 2-Methyl-2-Pentenal, Acetaldehyd, Aceton, Allylalkohol, Allyloxipropanol, DPG-Allylether, Ethylbenzol, Ethylen, Ethylenoxid, Methanol, Propionaldehyd, Propylenoxid und/oder Toluol) enthält. In dieser Phase ist Wasser gelöst, deren Umfang von der Art des Polyetherpolyols, welches vom Molgewicht bzw. der C-Kettenlänge und dem Anteil und Verteilung an Ethylenoxid und Propylenoxid abhängt. Die wässrige Phase enthält Salz, das Ionen der Alkali- und Erdalkaligruppe, z. B. Li, Na, K, Be, Mg, Ca, Sr, Ba enthalten kann und bei der Aufarbeitung bzw. Neutralisation mit Säuren z. B. H₂SO₄, HCl, H₃PO₄, HNO₃ oder CO₂ anfällt. Weiterhin kann zur Verbesserung der Phasentrennung ein mit Wasser nicht mischbares aromatisches oder aliphatisches Lösungsmittel (wie z. B. Toluol oder Hexan) eingesetzt werden

Im konkreten Fall zur Herstellung von Polycarbonat, Copolycarbonat oder Polyestercarbonat nach dem so genannten Phasengrenzflächenverfahren (vgl. Phasengrenzflächenverfahren zur Polycarbonatherstellung, s. Ullmann's Encyclopidia of Industrial Chemistry 2002 Wiley-VCH Verlag) werden Dihydroxyarylalkane in Form ihrer Alkalisalze mit Phosgen in heterogener Gegenwart von anorganischen Basen wie Natronlauge und einem organischen Lösungsmittel (wie z. B. Chlorbenzol und/oder Methylenchlorid), in dem das Produkt Polycarbonat gut löslich ist, umgesetzt. Während der Reaktion ist die wässrige Phase in der organischen Phase verteilt und nach der Reaktion wird die organische, Polycarbonat enthaltende Phase mit einer wässrigen Flüssigkeit gewaschen und mit Säuren (z. B. HCl) neutralisiert, wobei unter anderem Elektrolyte (wie z. B. Natruimchlorid, Natriumcarbonat, eventuell Natriumsulfat) und Spuren unreagierter Rohstoffe (wie z. B. Phenol, Isooctylphenol, Ethylpiperidin und Bisphenol) entfernt werden, und die Waschflüssigkeit anschließend abgetrennt. Üblicherweise wird die wässrige Phase durch Sprühtrocknung, Eindampfextrusion, Ausfällen des Polycarbonats, Zentrifugieren (s. EP 264 885 A2) oder durch Faserkoalezenz (s. DE 19 510 061A1) abgetrennt.

Im Allgemeinen stellen Salze sich als Verunreinigung dar und müssen vom organischen Produkt (beispielsweise Polycarbonat oder Polyetherpolyol) abgetrennt werden.

Die Aufgabe kann durch ein neues Filtrationsverfahren gelöst werden, bei dem sowohl Wasser als auch Elektrolyte (Laugen, Säuren, Salze) zurückgehalten und lediglich die organische Phase gegebenenfalls mit Resten an physikalisch gelösten Wasser durch organophile Filter hindurchgelassen werden.

Filtrationsverfahren werden üblicherweise zur Fest-Flüssig-Trennung, wie z. B. zur Abtrennung von Partikeln eingesetzt. Sie haben den Nachteil, dass sie nicht in der Lage sind, fein verteilte Dispersionen oder Emulsionen von Wasser und Salz in organischen Lösungsmitteln (Produkten) voneinander zu trennen. Übliche Membranfiltrationsverfahren mittels polymerer Membranen (z. B. aus Polyethersulfon, Polysulfon, Polyamid, Celluloseacetat) trennen in der Regel die wässrige Phase von der organisch-wässrigen Mischung ab, so dass die abfiltrierte wässrige Phase den Elektrolyten enthält und in der Regel das gewünschte Produkt ist und die organische Phase oftmals Restelektrolyte enthält und daher gesondert aufgearbeitet werden muss oder entsorgt wird. Weiterhin haben sie den Nachteil einer geringen chemischen Stabilität gegenüber organischer Verbindungen und eine hohe Temperaturempfindlichkeit.

Eine weitere Möglichkeit die Phasen voneinander zu trennen ist der Einsatz von Membranen die das Wasser selektiv abtrennen (siehe Verfahren zur Pervapoation oder Dampfpermeation; s. Melin, Rautenbach - Membranverfahren - Springer Verlag 2004). Hierbei penetriert die wässrige Phase durch die Membran und die organische Phase wird zurückgehalten. Bei salzhaltigen Lösungen kommt es zu einem Ausfallen der Salze im Bereich der Trenn- und Stützschicht dieser Membranen, so dass diese Membranen zur Verblockung neigen. Gleichzeitig zeigen diese Membranen einen geringen Permeationsfluss.

Des weiteren wird von Feng et al. der Einsatz von ultrahydrophoben beschichteten Sieben zur Trennung von Dieselöl und Wasser beschrieben. (Feng, L., Zhang, Z., Mai, Z., Ma, Y., Liu, B., Jiang, L., & Zhu, D., A Super-Hydrophobic and Super-Oleophilic Coating Mesh Film for the Separation of Oil and Water. Angewandte Chemie, 116 (2004) 2046). Feng et al. beschreibt neben der Herstellung der ultrahydrophoben beschichteten Sieben den Einfluss der Siebmaschenweite auf die Hydrophobie der Siebe. Die Beschreibung der Ultrahydrophobie erfolgt anhand Wassertropfen und Dieselöltropfen. Als mögliches Einsatzgebiet wird die Trennung von Dieselöl-Wasser beschrieben, wobei ausdrücklich die Flüssigkeiten in nicht emulgierter Form vorliegen. Die Arbeiten von Feng beziehen sich ausschließlich auf die Herstellung der Ultrahydrophoben-Siebe zur Trennung von Wasser und Dieselöl. Die Trennung von salzhaltigen bzw. elektrolythaltigen wässrigen Phasen wird nicht beschrieben und durchgeführt.

Aufgabe der vorliegenden Erfindung war es daher, die Trennung einer von Elektrolyt befreiten organischen Phase von einer elektrolythaltigen (salzhaltigen) wässrigen und organischen Phase zu ermöglichen bzw. zu verbessern.

Es wurde nun gefunden, dass sich diese Aufgabe durch das nachfolgend beschriebene spezielle Trennverfahren lösen lässt. Im Wesentlichen ist dies ein Trennverfahren über ein Trennmittel mit hydrophobem bzw. hydrophobiertem Material, welches als Membran oder Gitter ausgebildet sein kann, mittels derer die wässrige Phase sowie Elektrolyte (Salze, Laugen, Säuren) zurückgehalten werden können, während die organische Phase durch das Material (Membran, Gitter) permeiert. Hierdurch konnte eine weitgehende Trennung der elektrolythaltigen wässrigen von der organischen Phase erreicht werden.

Gegenstand der Erfindung ist ein Verfahren zur Abtrennung einer organischen Phase aus einem Gemisch aus elektrolythaltigem Wasser und einer organischen Lösung, dadurch gekennzeichnet, dass das zu trennende Gemisch über ein hydrophobes poröses Trennmittel, insbesondere eine organophile Membran oder ein textiles Flächengebilde oder eine Lochplatte, insbesondere ein Flächengebilde aus Metall oder Kunststoff mit hydrohober Beschichtung bzw. hydrophober Oberfläche, geleitet wird, ein Teil der organischen Phase des Gemisches durch das Trennmittel permeiert, während die Elektrolyt enthaltende wässrige Phase vollständig oder zu einem großen Anteil zusammen mit dem Rest der organischen Phase zurückgehalten wird.

Ein organophiles (hydrophobes) Trennmittel im Sinne der Erfindung weist eine Oberfläche auf, die einen Wasserrandwinkel an Luft Normalbedingungen von > 90° hat.

Bevorzugt ist das Trennmittel eine organophile Membran und bestehend aus Keramik, Metall, Polymer, Glas oder Verbundmaterialen aus Keramik und Glas und/oder Polymer und Keramik oder Metall, welche entweder eine Beschichtung mit organophilem Material, z. B. Perfluorierten Polymeren (Polytetrafluorethylen (PTFE), Polyvinylidenfluorid (PVDF)), hydrophoben Polymeren (z. B. Polypropylen PP, Poly(methylsilsesquioxan) PMSSQ) oder eine Beschichtung unter Verwendung von Beschichtungsmitteln wie Isocyanaten, Silanen oder Hybrid-Co-Polymeren aufweist.

Besonders bevorzugt besteht die organophile Membran aus Keramik, Metall, Polymer, Glas oder entsprechenden Verbundmaterialen aus Keramik und Glas oder aus Polymer und Keramik oder Metall, die ohne weitere Modifikation (z. B. Beschichtung) eine ausreichende Hydrophobie (bzw. Organophilie) aufweist.

Ein besonders bevorzugtes Verfahren ist dadurch gekennzeichnet, dass die organophile Membran eine keramische hydrophobisierte Membran ist.

Als keramische Membranen werden insbesondere Membranen auf Basis von Al₂O₃, ZrO₂, TiO₂, SiC (Siliziumcarbid) und Kombinationen dieser Verbindungen eingesetzt.

Die organophile Membran weist insbesondere eine Porenweite von 0,05 µm bis 10 µm, besonders bevorzugt von 0,1 µm bis 5 µm auf.

Elektrolyt im Sinne der Erfindung sind in Wasser lösliche Salze, Laugen oder Säuren insbesondere anorganische Verbindungen.

Ein besonders bevorzugtes Verfahren/Trennmittel ist dadurch gekennzeichnet, dass die hydrophobe poröse Membran eine organophile Polymermembran ist. Als Polymermembranen werden insbesondere Membranen auf Basis von (PP Polypropylen), (PVDF Polyvinylidenfluorid), (PTFE Polytetrafluorethylen), Silikone und Kombinationen dieser Verbindungen eingesetzt.

Die organophile Polymermembran weist Poren mit insbesondere einer Porenweite von 0,05 µm bis 10 µm, besonders bevorzugt von 0,1 µm bis 5 µm auf.

Ein alternatives bevorzugtes Verfahren ist dadurch gekennzeichnet, dass als hydrophobes poröses Trennmittel ein gewebtes Metallgitter mit hydrophober Beschichtung eingesetzt wird, bevorzugt weist das Metallgitter eine Maschenweite < 500 µm, bevorzugt < 50 µm, ganz besonders bevorzugt <5 µm auf.

Ein für eine hydrophobe Beschichtung besonders bevorzugt geeignetes organophiles Material ist ausgewählt aus einer Liste der folgenden Materialien jedoch grundsätzlich nicht darauf begrenzt: gelöstes PTFE (z.B. Teflon® AF), PTFE-Dispersion, Isocyanate, organische und/oder fluorierte Polymere oder Latex, Hybrid-Co-Polymere, Fettsäure, Silane, Lacke, Silikone, Silikonöle oder Polysilsesquioxane, oder aus einem Plasma-Prozess aufgebrachte, organische und/oder fluorierte Ablagerungen, oder einer Mischung unterschiedlicher vorgenannter Beschichtungsagenzien. Die Hybrid-Co-Polymere sind insbesondere Diblock-Co-Polymere mit einem am Metallgitter gut anbindenden und einem nicht anbindenden, hydrophoben Polymerblock, wobei der anbindende Block in einer bevorzugten Ausführung aus einem Polysilsesquioxan, besonders bevorzugt Poly(methylsilsesquioxan) oder Poly(stearylsilsesquioxan) besteht, und der nicht anbindende, hydrophobe Polymerblock in einer besonders bevorzugten Ausführung ausgewählt sein kann aus, aber nicht limitiert ist auf, Polystyrol, Polymethylacrylat, Polyethylhexylacrylat, Polymethylmethacrylat, Polydecylmethacrylat

Ein besonders bevorzugtes Verfahren ist dadurch gekennzeichnet, dass als hydrophobes poröses Trennmittel ein hydrophobes textiles Flächengebilde, insbesondere ein Gewebe, Gestrick oder Filz aus hydrophoben Kunststoff (insbesondere Polypropylen, PTFE oder PVDF) ohne weitere Modifikation eingesetzt wird. Bevorzugt weist das hydrophobe textile Flächengebilde eine Poren-/Maschenweite < 500 µm, bevorzugt < 50 µm, ganz besonders bevorzugt <5 µm auf.

In einem ganz besonders bevorzugten Verfahren ist die hydrophobe Beschichtung durch das Aufbringen einer glatten, hydrophoben Beschichtung auf eine vorher aufgeraute Trennmitteloberfläche bewirkt.

Das Verfahren wird bevorzugt angewendet, wenn das zu trennenden Gemisch eine emulsionsartige Dispersion, bestehend aus Wasser, Elektrolyt und Organika, insbesondere Elektrolyte der Alkali- und/oder Erdalkalimetalle, und Organika aus Polymeren, Aromaten und/oder Aliphaten ist.

Das Verfahren wird besonders bevorzugt angewendet, wenn das zu trennende Gemisch eine emulsionsartige Dispersion von in organischem Lösungsmittel gelöstem Polycarbonat, gegebenenfalls Salzen der Alkali- und Erdalkalimetalle, insbesondere Natrium, Kalium, Magnesium, Calcium und Wasser ist.

Das Verfahren wird auch bevorzugt angewendet, wenn das zu trennende Gemisch eine Mischung aus einem Polyol gegebenenfalls mit Lösungsmitteln (insbesondere aromatische oder aliphatische Kohlenwasserstoffe, z. B. Toluol oder Hexan) sowie einem Salz der Alkali- und Erdalkalimetalle, wie Natrium, Kalium, Magnesium, Calcium und Wasser ist.

Die hydrophoben Trennmittel im erfindungsgemäßen Verfahren zeigten eine hohe Abtrennrate von Salz und Wasser aus dem Organikgemisch, gepaart mit einem hohen Permeationsfluss und einer hohen Beständigkeit gegen organische Lösungsmittel.

In dem zu trennenden Gemisch enthaltene Salze wurden zusammen mit der wässrigen Phase zurückgehalten und ließen sich dann problemlos entfernen.

Ein hydrophobiertes Gitter besteht besonders bevorzugt aus form- und druckstabilen Edelstählen besonders bevorzugt aus form- und druckstabilen austenitischen nichtrostenden Stählen (1.4404, 1.4571). Das Sieb ist bevorzugt mit einer Maschenweite < 500 µm, insbesondere bevorzugt mit einer Maschenweite < 100 µm, besonders bevorzugt mit einer Maschenweite 20 µm und ganz besonders bevorzugt mit einer Maschenweite < 5 µm anzuwenden.

Die Beschichtung des Gitters besteht besonders bevorzugt aus einem hydrophoben Material, insbesondere bevorzugt aus unpolaren Polymeren, Hybrid-Co-Polymeren, Polysilsesquioxanen oder Silanen, besonders bevorzugt aus Polysilsesquioxan-Block-Polymeren oder Polytetrafluorethylen. Die Aufbringung des hydrophoben Materials kann dabei durch Aufsprühen, Lösungsbenetzung, Sublimation oder andere geeignete Verfahren erfolgen.

Im einfachsten Fall erfolgt die Modifizierung des Gitters durch Aufbringen eines gelösten hydrophoben Materials mit nachfolgender Verdampfung des Lösungsmittels, bevorzugt durch Verdampfen einer aufgebrachten Polymerlösung, bevorzugt einer Polysilsesquioxan-Block-Co-Polymerlösung, Polytetrafluorethylenlösung oder i-Polypropylenlösung (Erbil, H. Y., Demirel, A. L., Avcinodot, Y., & Mert, O., Transformation of a Simple Plastic into a Superhydrophobic Surface. Science, 299, no. 5611, (2003) 1377-80), besonders bevorzugt einer Lösung von Poly(methylsilsesquioxan)-block-poly(methylmethacrylat).

Alternativ kann die Modifizierung des Gitters auch durch die kovalente Anbindung von hydrophoben Silanen erfolgen (Nakajima, A., Abe, K., Hashimoto, K., & Watanabe, T., Preparation of hard super-hydrophobic films with visible light transmission. Thin Solid Films, 376, no. 1-2, (2000) 140-3; Nakajima, A., Fujishima, A., Hashimoto, K. & Watanabe, T., Preparation of Transparent Superhydrophobic Boehmite and Silica Films by Sublimation of Aluminum Acetylacetonate, Advanced Materials, 11, no. 16, (1999) 1365); Bico, J., Marzolin, C., & Quere, D., Pearl drops, Europhys Lett, 47, no. 2, (1999) 220).

Eine weitere Möglichkeit zur Modifizierung der Gitter ist die Behandlung der Metallgitter durch Aufsprühen einer Polymerdispersion, z. B. einer Polytetrafluorethylen-Dispersion in Wasser und anschließendes Sintern bei 320 °C bzw. einer Poly(methylsilsesquioxan)-block-poly(methylmethacrylat)-Lösung in THF und anschließendes Sintern bei 130 °C.

Die Hydrophobie der Schicht kann zusätzlich durch Erhöhung der Rauhigkeit der Oberfläche erhöht werden, bevorzugt durch Sandstrahlen oder Anätzen des Metallgitter vor der Beschichtung, bevorzugt durch Zugabe von Nanopartikeln in die Beschichtung, oder bevorzugt durch anschließende Plasmabehandlung der Beschichtung (Morra, M., Occhiello, E., & Garbassi, F., Contact angle hysteresis in oxygen plasma treated poly(tetrafluoroethylene) Langmuir, 5, no. 3, (1989) 872).

Nach Modifizierung ergaben sich Wasserrandwinkel auf der Schicht (gemessen gegen Luft) > 90°, bevorzugt > 120°, besonders bevorzugt > 140°.

Eine weitere Möglichkeit zur Lösung der Aufgabe ist die Verwendung von hydrophobierten keramischen Membranen. Hierbei tritt ebenfalls die organische Phase durch die Membran und die elektrolythaltige wässrige Phase wird zurückgehalten. Die Elektroyte/Salze verbleiben im Wasser, so dass es daher zu keiner Verblockung dieser Membran durch Salze kommt, wie Sie bei hydrophilen wasserselektiven Membranen entsteht. Die Herstellung hydrophobierter keramischer Membranen ist beispielsweise in DE 10 308 110 A1 unter Verwendung von Fluorsilanen beschrieben. Gleichzeitig ist es denkbar hydrophobe Membranen aus SiO₂ oder Vycor Glas herzustellen. Die verwendeten hydrophobierten keramischen Membranen können eine Porengröße von 0,05 bis 10 µm, besonders bevorzugt zwischen 0,1 bis 5 µm aufweisen.

Eine weitere Möglichkeit zur Lösung der Aufgabe ist die Verwendung von hydrophoben Polymermembranen aus PP, PVDF oder PTFE welche eine ausreichende chemische Stabilität gegen die im jeweiligen verwendeten organischen Phasen aufweisen. Hierbei tritt ebenfalls die elektrolytfreie organische Phase durch die Membran und die elektrolythaltige wässrige Phase wird zurückgehalten. Besonders geeignet sind Membranen aus Polypropylen PP mit einer Porengröße von 0,05 bis 3 µm, besonders bevorzugt von 0,1 bis 1 µm.

Unter Anwendung des erfindungsgemäßen Verfahrens konnte z. B. die Abtrennung eines elektrolytfreien organischen Polyetherpolyols aus einer Elektrolyt und Wasser enthaltenden Polyetherpolyollösung in einer einzigen Trennstufe bis zur physikalische Löslichkeit des Wassers in dem eingesetzten Polyol durchgeführt werden. Die salzhaltige wässrige Phase, als disperse Phase war damit nahezu vollständig abgetrennt. Gleichzeitig konnte das Salz (K₂SO₄) bis 99,9% zurückgehalten werden. Die Salzrückhaltung konnte auch beobachtet werden, wenn ein mit Wasser fast vollständig mischbarer Polyether eingesetzt wurde. Hierbei wurde die im Retentat verbleibende Phase mit Salz angereichert.

Unter Anwendung des erfindungsgemäßen Verfahrens konnte z.B. auch die Abtrennung einer salzfreien organischen Polycarbonatlösung aus einer Elektrolyt und Wasser enthaltenden Polycarbonatlösung, in einer einzigen Trennstufe, auf einen Wasseranteil von bis zu 0,1 % bzw. bis zur physikalischen Löslichkeit in der abgetrennten Polycarbonatlösung durchgeführt werden. Gleichzeitig konnte das Salz (NaCl) mit bis zu 99,9%, bezogen auf den Ausgangswert der Polycarbonatlösung zurückgehalten werden. Die elektrolythaltige wässrige Phase, als disperse Phase war damit nahezu vollständig von der organischen Phase abgetrennt.

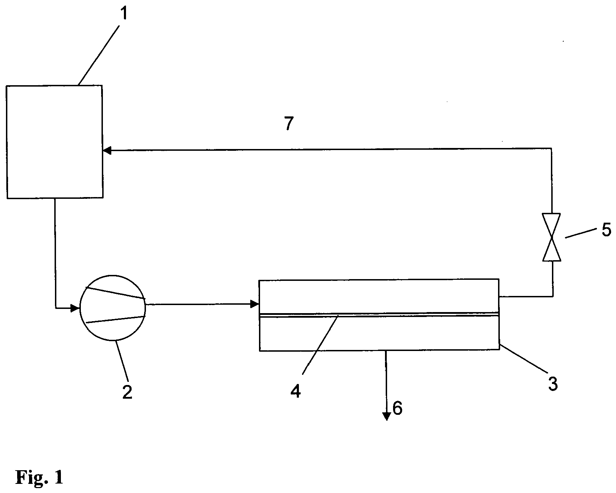

Die Erfindung wird nachstehend beispielhaft an Hand der Figur 1 näher erläutert. Hierin zeigen:
- 1: Rührkessel/Vorlage
- 2: Kreislaufpumpe
- 3: Membranmodul für Sieb/ Membran
- 4: hydrophobes Sieb/ Membran
- 5: Ventil
- 6: Organische Phase (Permeat)
- 7: wässrige Phase (Retentat)

### Beispiele:

Zur Durchführung des erfindungsgemäßen Verfahrens wird eine Crossflow Filtrationszelle 3 mit einem hydrophoben Trennmittel 4 eingesetzt. Die zu trennende Lösung wird im Rührkessel 1 vorgelegt und im Kreislauf durch eine Pumpe 2 über das Trennmittel (Membran/Sieb) 4, mit einer vorgegebenen Überströmgeschwindigkeit gepumpt. Durch ein Ventil 6 kann eine entsprechende transmembrane Druckdifferenz (TMP = (p_{Feed}+p_{Retentat})/2-p_{Permeat}) eingestellt werden, so dass die organische Lösung, welche eine geringere Wasser- und Salzkonzentration im Permeat 6 aufweist als im Retentat 7, durch das Trennmittel (Membran/Sieb) permeiert (Permeat). Die an Organik abgereicherte elektrolyt-/salzhaltige wässrige und organische Phase (Retentat 7) wird in Rührkessel 1 zurückgeführt. Zur analytischen Bestimmung des Wasserwertes wird eine Karl-Fischer-Titration durchgeführt. Die Bestimmung der jeweiligen Elektrolyt-(Salz)-konzentration erfolgt durch Titration des Gesamtbasengehaltes oder durch Atomabsorptionsspektroskopie (AAS). Die Nachweisgrenze für Na liegt bei 15 ppb (µg/kg).

### Beispiel 1 Herstellung eines hydrophobisierten Siebes

Für die Herstellung eines ultrahydrophoben Siebes wird ein austenitischer Stahl mit der Bezeichnung 1.4404 als Basis verwendet. Die absolute Filtereinheit beträgt 5 µm (DTW 4). Das Metallsieb wird in einem 1:1-Gemisch bestehend aus Ethanol und n-Hexan gereinigt. Anschließend wird die Oberfläche mit einem Gemisch aus H₂O, H₂O₂ und Ammoniakwasser bei 80°C angeätzt. Nach dem Abspülen mit Wasser erfolgt die Beschichtung mit einer 5 %igen Teflon®-Dispersion 30-N. Zur Trocknung der PTFE-Dispersion wird das Sieb bei 320°C für 30 Minuten gesintert. Der Wasserrandwinkel eines derartig beschichteten Gitters beträgt 140° ± 5°. Dabei wurde der so genannte statische Randwinkel eines etwa 10 µl großen Tropfens destillierten Wassers auf dem Gitter bei Raumtemperatur gemessen.

### Beispiel 2

Wie in der allgemeinen Versuchsdurchführung beschrieben wird ein hydrophobes Gitter wie nach Beispiel 1 hergestellt (Sieb 06 DTW Maschenweite 4 bis 5 µm, hydrophobisiert mit 5 % Teflon® N 30), in die oben beschriebene Testapparatur eingesetzt. Die Filtrationsfläche beträgt 0,0044 m². Zum Einsatz kommt eine Polyetherpolyol-Lösung (Trimethylolpropan gestarteter Polyether mit Trifunktionalität und einer OH-Zahl von 45, Seitenketten mit 16 % Ethylenoxideinheiten und vorwiegend sekundären OH-Gruppen). Die Tabelle 1 zeigt das Ergebnis der Phasentrennung.

**Tabelle 1**

| **Kessel** | **ÜberStrömung** | **TMP** | **Permeatfluss** | **Feed** | **Permeat** |
|---|---|---|---|---|---|
| **[°C]** | **[m/s]** | **[bar]** | **[kg/m²hbar]** | **[% H₂O]** | **[% H₂O]** |
| 78,7 | 1,5 | 0,06 | 403,4 | 7,8 | 5,6 |
| 78,7 | 1,5 | 0,06 | 375,0 | 8,1 | 5,7 |
| 79,0 | 1,5 | 0,07 | 761,5 | 8,1 | 5,6 |
| 78,9 | 1,5 | 0,18 | 542,9 | 8,1 | 5,6 |
| 79,0 | 1,5 | 0,22 | 458,7 | 8,2 | 5,6 |
| 79,0 | 1,5 | 0,36 | 265,2 | 8,1 | 5,6 |
| 79,3 | 1,5 | 0,36 | 238,9 | 8,2 | 5,9 |
| 78,7 | 1,5 | 0,40 | 211,9 | 8,2 | 5,9 |

### Beispiel 3

Wie in der allgemeinen Versuchsdurchführung beschrieben wird ein hydrophobes Gitter wie nach Beispiel 1 hergestellt (Sieb 06 DTW Maschenweite 4 bis 5 µm, hydrophobisiert mit 5 % Teflon N 30) mit einem eingegossenen PTFE-Dichtrand, in die oben beschriebene Testapparatur eingesetzt. Die Filtrationsfläche beträgt 0,0044 m². Die Versuchsdurchführung erfolgte analog Beispiel 2. Zum Einsatz kommt eine Polyetherpolyol-Lösung (Produkt wie in Beispiel 2). Die Tabelle 2 zeigt das Ergebnis der Phasentrennung.

**Tabelle 2**

| **Kessel** | **ÜberStrömung** | **TMP** | **Permeatfluss** | **Feed** | **Permeat** | **Feed** | **Permeat** |
|---|---|---|---|---|---|---|---|
| **[°C]** | **[m/s]** | **[bar]** | **[kg/m²hbar]** | **[ppm KOH]** | **[ppm KOH]** | **[% H₂O]** | **[% H₂O]** |
| 114,3 | 1,5 | 0,3 | 1246 | 1826 | 0,1 | 4,0 | 4,0 |
| 114,9 | 1,5 | 0,5 | 766 | 1859 | 5,8 | 4,0 | 3,9 |
| 115,5 | 1,5 | 1,0 | 464 | 2194 | 4,2 | 4,1 | 3,8 |

### Beispiel 4

Die Durchführung erfolgt analog Beispiel 2, jedoch unter Verwendung einer keramischen organophilen Membran. Die Membran, ein sogenanntes Einkanalrohr mit einem rohrförmigen Kanal, mit einem Innendurchmesser von 6 mm und einer Länge von 250 mm und einer aktiven inneren Filtrationsfläche von 0,005 m², besteht aus Al₂O₃ und wurde nach der Beschreibung in der Patentschrift DE10 308 110 A1 silanisiert. Der Porendurchmesser der Membran beträgt 3 µm.

Durch kontinuierliches Umpumpen der Feedlösung (Produkt wie in Beispiel 2) über die Innenseite der eingesetzten Membran konnte ein klares Polyetherpermeat abgezogen werden, wobei die Wasserkonzentration im Bereich der physikalischen Löslichkeit liegt. Entsprechend konnte ein Ansteigen der Wasserkonzentration im Feed erreicht werden, was einer Aufkonzentrierung der wässrigen Phase im Kreislauf entspricht.

Die Tabelle 3 zeigt die Phasentrennung des Polyethers mittels einer hydrophoben Keramik-Membran mit einer Porengröße von 3 µm.

**Tabelle 3**

| **Kessel** | **Überströmung** | **TMP** | **Permeatfluss** | **Feed** | **Permeat** |
|---|---|---|---|---|---|
| **[°C]** | **[m/s]** | **[bar]** | **[kg/m²hbar]** | **[% H₂O]** | **[% H₂O]** |
| 77,0 | 1,0 | 0,65 | 47,1 | 8,5 | 5,9 |
| 81,0 | 2,0 | 1,36 | 56,3 | 8,3 | 4,8 |
| 79,6 | 3,0 | 2,11 | 64,5 | 8,4 | 5,2 |
| 80,1 | 3,0 | 2,15 | 63,3 | 8,6 | 4,7 |
| 80,1 | 3,0 | 2,11 | 56,0 | 8,8 | 5,2 |
| 80,1 | 3,0 | 2,11 | 49,4 | 9,1 | 4,7 |
| 80,1 | 3,0 | 2,1 | 47,1 | 10,0 | 4,6 |
| 80,1 | 3,0 | 2,13 | 45,7 | 11,1 | 4,5 |
| 80,1 | 3,0 | 2,21 | 41,5 | 11,3 | 4,9 |

### Beispiel 5

In der oben beschriebenen Anlage wurde eine mit Salzsäure (HCl) neutralisierte Polycarbonatlösung (PC-Molekulargewicht M_{w} ca. 18 000 bis 19 000 g/mol), kommend aus einer Waschstufe aus dem Grenzflächenverfahren zur Herstellung von Polycarbonat, bestehend aus Polycarbonat, Monochlorbenzol, Methylenchlorid und Natrium-Salzen (NaCl) sowie geringen Mengen an Wasser eingesetzt. Der Natriumgehalt in der Feedlösung, lag zu Versuchsbeginn bei 4,3 ppm (4300 ppb). Die Polycarbonatphase war durch die wässrige Emulsion eingetrübt. Durch den Einsatz des erfindungsgemäßen Verfahrens ist es gelungen eine salzfreie organische Lösung mittels einer organophilen keramischen Membran zu separieren. Die Membran, ein so genanntes Multikanalrohr mit 7 rohrförmigen Kanälen, mit einem Innendurchmesser der Kanäle von 6mm und einer Länge von 250 mm und einer aktiven inneren Filtrationsfläche von 0,03 m², besteht aus Al₂O₃ und wurde nach der Beschreibung in der Patentschrift DE 10 308 110 A1 silanisiert. Der Porendurchmesser der Membran beträgt 1 µm. Die Ergebnisse des Versuches sind in Tabelle 4 abgebildet und zeigen deutlich die Verminderung des Natriumgehaltes der Polycarbonatlösung. Alle Permeatproben sind klar und weisen augenscheinlich keine Trübung auf.

**Tabelle 4**

| **Kessel** | **Überströmung** | **TMP** | **Permeatfluss** | **Permeat** |
|---|---|---|---|---|
| **[°C]** | **[m/s]** | **[bar]** | **[kg/m²hbar]** | **[ppb Na]** |
| 36,8 | 1,2 | 2,06 | 55,8 | 20 |
| 37,5 | 1,1 | 2,10 | 45,9 | <15 |
| 35,9 | 1,1 | 2,15 | 28,6 | <15 |

### Beispiel 6

In der wie oben beschriebenen Anlage (Fig. 1) wurde eine mit Salzsäure (HCl) neutralisierte Polycarbonatlösung, kommend aus einer Waschstufe aus dem Grenzflächenverfahren, bestehend aus Polycarbonat, Monochlorbenzol, Methylenchlorid und Natrium-Salzen (NaCl) sowie geringen Mengen an Wasser eingesetzt. Der Natriumgehalt in der Feedlösung, lag zu Versuchsbeginn bei 0,8 ppm (800 ppb). Der Wassergehalt wurde zu Versuchsbeginn mit 0,22 Gew.-% bestimmt. Die Polycarbonatphase war durch die wässrige Emulsion eingetrübt. Durch den Einsatz des erfindungsgemäßen Verfahrens ist es gelungen eine salzfreie organische Lösung mittels einer organophilen keramischen Membran bestehend aus silanisiertem Al₂O₃ wie in Beispiel 3 beschrieben, jedoch mit einer Porengröße von 0,8 µm, zu separieren. Die Ergebnisse des Versuches sind in Tabelle 5 abgebildet und zeigen deutlich die Verminderung des Natriumgehaltes und Wassergehaltes der Polycarbonatlösung. Alle Permeatproben sind klar und weisen augenscheinlich keine Trübung auf.

**Tabelle 5**

| **Kessel** | **Überströmung** | **TMP** | **Permeatfluss** | **Permeat** | **Permeat** |
|---|---|---|---|---|---|
| **[°C]** | **[m/s]** | **[bar]** | **[kg/m²hbar]** | **[ppb Na]** | **[% H₂O]** |
| 27,9 | 0,8 | 2,08 | 36,9 | 20 | 0,09 |
| 28,6 | 0,7 | 2,02 | 31,0 | <15 | 0,08 |
| 27,4 | 0,7 | 2,08 | 14,2 | <15 | 0,10 |

### Beispiel 7

In der wie oben beschriebenen Anlage wurde eine noch alkalische bzw. elektrolythaltige (nicht neutralisierte) Polycarbonatlösung, kommend aus einer Waschstufe aus dem Grenzflächenverfahren, bestehend aus Polycarbonat, Monochlorbenzol, Methylenchlorid und Natriumhydroxyd sowie geringen Mengen an Wasser eingesetzt. Der Natriumgehalt in der Feedlösung, lag zu Versuchsbeginn bei 2,9 ppm (2900 ppb). Der Wassergehalt wurde zu Versuchsbeginn mit 5,4 Gew.-% bestimmt. Die Polycarbonatphase war durch die wässrige Emulsion eingetrübt. Durch den Einsatz des erfindungsgemäßen Verfahrens ist es gelungen eine elektrolytfreie organische Polycarbonatlösung mittels einer organophilen PP-Polymermembran zu separieren. Die verwendete PP-Membran ist rohrförmig mit einem Innendurchmesser von 5,5 mm und einem Außendurchmesser von 8,5 mm, einer Länge von 250 mm und einer aktiven inneren Filtrationsfläche von 0,004 m². Der Porendurchmesser der Membran beträgt 0,2 µm. Die Ergebnisse des Versuches sind in Tabelle 6 abgebildet und zeigen deutlich die Verminderung des Natriumgehaltes und des Wassergehaltes in der abgetrennten Polycarbonatlösung. Alle Permeatproben sind klar und weisen augenscheinlich keine Trübung auf.

**Tabelle 6**

| **Kessel** | **Überströmung** | **TMP** | **Permeatfluss** | **Permeat** | **Permeat** |
|---|---|---|---|---|---|
| **[°C]** | **[m/s]** | **[bar]** | **[kg/m²hbar]** | **[ppb Na]** | **[% H₂O]** |
| 33,3 | 3,14 | 0,70 | 64,0 | <15 | 0,09 |
| 33,2 | 3,14 | 0,67 | 23,3 | <15 | 0,1 |
| 33,1 | 3,14 | 1,18 | 8,6 | 20 | 0,1 |
| 33,1 | 3,14 | 1,18 | 3,8 | <15 | 0,1 |

### Beispiel 8

THF wurde über Natrium/Benzophenon unter Stickstoff destilliert, alle verwendeten Monomere unter vermindertem Druck vor der Polymerisation umkondensiert. CuBr wurde 24h mit Essigsäure gerührt, abfiltriert, mit Methanol gewaschen und im Vakuum getrocknet.

**Synthese von 2-Bromisobuttersäure-pent-4-enyl-ester (1).** 160 mmol (13,9 g) Penten-4-ol und 50 mL Chloroform wurden in ein Kolben gegeben und auf 0 °C abgekühlt. Langsam wurden 160 mmol (36,8 g) 2-Bromisobuttersäurebromid in 20 mL Chloroform unter Kühlung zugegeben und die dadurch entstandene Lösung 4 h bei Raumtemperatur gerührt. Die Reaktionslösung wurde dreimal mit Wasser gewaschen, über MgSO₄ getrocknet und anschließend eingedampft. Das Produkt wurde im Hochvakuum destilliert. Siedepunkt 50 °C bei 3,3*10⁻² mbar. Ausbeute: 34,79 g (148 mmol; 92,5%)
¹H NMR (CDCl₃): (ppm) = 5,74 (m,1H); 4,96 (m, 2H); 4,12 (t, 3J = 6,6 Hz, 2H); 2,10 (m, 2H); 1,87 (s, 6H); 1,72 (quin., 3J = 6,3 Hz, 2H)
¹³C NMR (CDCl₃): (ppm) = 171,34; 137,05; 115,35; 65,05; 55,66; 30,58; 29,73; 27,37. FD-Massenspektrum: 234,0 (27,8%); 235,0 (100%)
EA (%): berechnet: C = 45,98; H = 6,43; gemessen: C = 45,87; H = 6,43.

**Synthese des PMSSQ-Makroinitiators 2-Bromisobuttersäure-5-(trichlosilanyl)-pentyl-ester (2).** Methyltrimethoxysilan (MTMS) und (1) im Molverhältnis 20:1 wurden in 20 mL THF gelöst und mit 1000 mol % Wasser und 3 mol % HCl versetzt. Die Lösung wurde anschließend 3 h bei 0 °C gerührt, mit Diethylether extrahiert, mit Wasser gewaschen und über MgSO₄ getrocknet. Das Lösungsmittel wurde verdampft und das Produkt im Hochvakuum getrocknet.
¹H NMR (CDCl₃): (ppm) = 5,55 (br); 4,14 (br, 2H); 3,44 (s); 1,91 (br, 6H); 1,77 (br, 2H); 1,60 (br, 2H); 1,42 (br, 2H); 0,63 (br, 2H); 0,13 (br)
²⁹Si Festkörper NMR: (ppm) = -48,26 (T1); -57,23 (T2); -65,87 (T3)

**Synthese von Poly(methylsilsesquioxan)-Block-Poly(methylmethacrylat) (3) mittels Atomtransfer-Radical Polymerisation.** Der Makroinitiator (2) (0.5 g), CuBr und 2,2'-Bipyridin im Verhältnis 1:1:2, sowie Methylmethacrylat (2 g) wurden in 4 mL Dioxan dreimal entgast. Die Lösung wurde 8 h bei 55 °C gerührt, das Hybrid-Co-Polymer mit n-Heptan gefällt, anschließend zweimal aus THF in n-Heptan umgefällt und im Hochvakuum getrocknet.
¹H NMR (CDCl₃): (ppm) = 4,92 (br); 3,57 (br); 1,72 (br); 1,03 (br); 0,15 (br).

**Beschichtung:** Ein Gitter in Form eines Metallsiebs des Typs DTW4 von der Fa. Haver und Böcker von etwa 89 mm Durchmesser aus austenitischem Stahl der Bezeichnung 1.4404 wird als Basistrennmittel benutzt, von dem die absolute Filtereinheit (die nominale Porengröße) 5 µm beträgt. Etwa 10 mL einer Lösung des Hybrid-Co-Polymers PMSSQ-b-PMMA (3) in THF mit der Konzentration 2,5 mg/mL wird in eine Petri-Schale gegeben und das Sieb für 30 Min. in die Lösung horizontal eingetaucht, anschließend ebenfalls in der Petri-Schale für ca. 5 Min. mit THF gewaschen und dann möglichst freiliegend für 1h bei 130 °C im Trockenschrank getempert. Der Wasserrandwinkel eines derartig beschichteten Siebs beträgt 93 °. Dabei wurde der fortschreitende Randwinkel eines etwa 10 µL großen Tropfens destillierten Wassers auf dem Sieb bei Raumtemperatur gemessen.

### Beispiel 9

Die Durchführung erfolgt analog Beispiel 2, jedoch unter Verwendung des hydrophobierten Gitters aus Beispiel 8. Zum Einsatz kommt eine Polyetherpolyol-Lösung (Produkt wie in Beispiel 2). Die Tabelle 7 zeigt das Ergebnis der Phasentrennung.

**Tabelle 7**

| **Kessel** | **ÜberStrömung** | **TMP** | **Permeatfluss** | **Feed** | **Permeat** | **Feed** | **Permeat** |
|---|---|---|---|---|---|---|---|
| **[°C]** | **[m/s]** | **[bar]** | **[kg/m²h]** | **[ppm KOH]** | **[ppm KOH]** | **[% H₂O]** | **[% H₂O]** |
| 88,2 | 0,9 | 0,02 | 61,9 | 2051 | 27 | 8,6 | 4,1 |
| 89,1 | 0,9 | 0,04 | 50,2 | n.b. | 28 | n.b. | 4,2 |
| 89,5 | 0,91 | 0,02 | 85,2 | n.b. | 27 | n.b. | 3,9 |

## Patentansprüche

1. Verfahren zur Abtrennung einer organischen Phase aus einem Gemisch einer organischen Lösung und elektrolythaltigem Wasser, **dadurch gekennzeichnet, dass** das zu trennende Gemisch über ein hydrophobes poröses Trennmittel, insbesondere eine organophile Membran oder eine Lochplatte oder ein textiles Flächengebilde aus Metalldraht, oder insbesondere ein Gewebe, Gestrick, Filz mit hydrophober Beschichtung geleitet wird, ein Teil der organischen Phase des Gemisches durch das Trennmittel permeiert während die elektrolythaltige, wässrige Phase vollständig oder zu einem großen Anteil zusammen mit dem Rest der organischen Phase zurückgehalten wird.

2. Verfahren nach Anspruch 1, **dadurch gekennzeichnet, dass** das Trennmittel eine organophile Membran ist und aus Keramik, Metall, Polymer, Glas oder entsprechenden Verbundmaterialen aus Keramik und Glas oder aus Polymer und Keramik oder Metall besteht und eine Beschichtung mit hydrophobem Material aufweist.

3. Verfahren nach Anspruch 1 bis 2, **dadurch gekennzeichnet, dass** die organophile Membran aus Keramik, Metall, Polymer, Glas oder entsprechenden Verbundmaterialen aus Keramik und Glas oder aus Polymer und Keramik oder Metall besteht und ohne Beschichtung hydrophob ist.

4. Verfahren nach Anspruch 2 oder 3, **dadurch gekennzeichnet, dass** die organophile Membran eine keramische hydrophobisierte Membran ist, insbesondere auf Basis von Al₂O₃, TiO₂, ZrO₂ oder SiC.

5. Verfahren nach Anspruch 2 oder 3, **dadurch gekennzeichnet, dass** die organophile Membran aus einem organophilen Polymer besteht, insbesondere ausgewählt aus: Polypropylen, Polytetrafluorethylen oder Polyvinylidenfluorid.

6. Verfahren nach Anspruch 4 oder 5, **dadurch gekennzeichnet, dass** die organophile Membran eine Porenweite von 0,05 µm bis 10 µm, bevorzugt von 0,1 µm bis 5 µm aufweist.

7. Verfahren nach Anspruch 1 **dadurch gekennzeichnet, dass** als hydrophobes poröses Trennmittel ein gewebtes Metallgitter mit hydrophober Beschichtung eingesetzt wird und eine Beschichtung mit hydrophobem und/oder organophilem Material aufweist, ausgewählte aus einer Liste inklusive der folgenden Materialien jedoch nicht darauf begrenzt: Teflon® AF, PTFE-Dispersion, Isocyanaten, organischen und/oder fluorierten Polymeren oder Latex, Hybrid-Co-Polymere, insbesondere Diblock-Co-Polymere aus einem Polysilsesquioxan-Block, bevorzugt Poly(methylsilsesquioxan) oder Poly(stearylsilsesquioxan), und einem hydrophoben Polymerblock ausgewählt aus der Reihe Polystyrol, Polymethylacrylat, Polyethylhexylacrylat, Polymethylmethacrylat, Polydecylmethacrylat, Fettsäuren, Silanen, Lacken, Silikonen, Silikonölen oder Polysilsesquioxane, oder aus einem Plasma-Prozess aufgebrachten, organischen und/oder fluorierten Ablagerungen, oder einer Mischung unterschiedlicher Beschichtungsagenzien.

8. Verfahren nach Anspruch 7 **dadurch gekennzeichnet, dass** als hydrophobes poröses Trennmittel ein hydrophobes Sieb ohne weitere Modifikation der Oberfläche eingesetzt wird.

9. Verfahren nach Anspruch 7 oder 8 **dadurch gekennzeichnet, dass** das Metallgitter eine Maschenweite < 500 µm, bevorzugt < 5 µm aufweist.

10. Verfahren nach einem der Ansprüche 1 bis 9, **dadurch gekennzeichnet, dass** das zu trennende Gemisch eine emulsionsartige Dispersion von einem mit Wasser nicht mischbaren aromatischen oder aliphatischen organischen Lösungsmittel oder Polymer und Wasser und Elektrolyt ist.

11. Verfahren nach einem der Ansprüche 1 bis 9, **dadurch gekennzeichnet, dass** das zu trennende Gemisch eine emulsionsartige Dispersion von in organischem Lösungsmittel gelöstem Polycarbonat und Wasser und Elektrolyt ist.

12. Verfahren nach einem der Ansprüche 1 bis 9, **dadurch gekennzeichnet, dass** das zu trennende Gemisch eine Mischung aus Polyetherpolyol und Wasser und Elektrolyt ist.

13. Verfahren nach Anspruch 2 oder 4, **dadurch gekennzeichnet, dass** die hydrophobe Beschichtung durch das Aufbringen einer glatten, hydrophoben Beschichtung auf eine vorher aufgeraute Trennmitteloberfläche bewirkt ist.

14. Verfahren nach Anspruch 2 oder 4, **dadurch gekennzeichnet, dass** die hydrophobe Beschichtung durch das Aufbringen einer rauen, hydrophoben Beschichtung auf eine Trennmitteloberfläche bewirkt ist.

## Claims

1. Method for separating an organic phase from a mixture of an organic solution with electrolyte-containing water, **characterized in that** the mixture to be separated is passed over a hydrophobic porous separating means, especially an organophilic membrane or a perforated plate or a textile fabric formed from metal wire, or especially a weave, knit, felt having a hydrophobic coating, some of the organic phase of the mixture permeates through the separating means while the electrolyte-containing, aqueous phase is completely or substantially retained together with the rest of the organic phase.

2. Method according to Claim 1, **characterized in that** the separating means is an organophilic membrane and consists of ceramic, metal, polymer, glass or corresponding composite materials combining ceramic with glass or polymer with ceramic or metal and has a coating with hydrophobic material.

3. Method according to Claim 1 to 2, **characterized in that** the organophilic membrane consists of ceramic, metal, polymer, glass or corresponding composite materials combining ceramic with glass or polymer with ceramic or metal and is hydrophobic without coating.

4. Method according to Claim 2 or 3, **characterized in that** the organophilic membrane is a ceramic hydrophobicized membrane, especially based on Al₂O₃, TiO₂, ZrO₂ or SiC.

5. Method according to Claim 2 or 3, **characterized in that** the organophilic membrane consists of an organophilic polymer, especially selected from: polypropylene, polytetrafluoroethylene or polyvinylidene fluoride.

6. Method according to Claim 4 or 5, **characterized in that** the organophilic membrane has a pore size of 0.05 µm to 10 µm, preferably of 0.1 µm to 5 µm.

7. Method according to Claim 1, **characterized in that** the hydrophobic porous separating means employed is a woven metallic lattice having a hydrophobic coating and has a coating with hydrophobic and/or organophilic material selected from a list including but not being limited to the following materials: Teflon® AF, PTFE dispersion, isocyanates, organic and/or fluorinated polymers or latex, hybrid copolymers, especially diblock copolymers formed from a polysilsesquioxane block, preferably poly(methylsilsesquioxane) or poly(stearylsilsesquioxane), and a hydrophobic polymeric block selected from the series polystyrene, poly(methyl acrylate), poly(ethylhexyl acrylate), poly(methyl methacrylate), poly(decyl methacrylate), fatty acids, silanes, lacquers, silicones, silicone oils or polysilsequioxanes, or organic and/or fluorinated deposits applied out of a plasma process, or a mixture of different coating agents.

8. Method according to Claim 7, **characterized in that** the hydrophobic porous separating means used is a hydrophobic sieve without further modification of the surface.

9. Method according to Claim 7 or 8, **characterized in that** the metallic lattice has a mesh size < 500 µm, preferably < 5 µm.

10. Method according to any one of Claims 1 to 9, **characterized in that** the mixture to be separated is an emulsion type dispersion of a water-immiscible aromatic or aliphatic organic solvent or polymer and water and electrolyte.

11. Method according to any one of Claims 1 to 9, **characterized in that** the mixture to be separated is an emulsion type dispersion of polycarbonate dissolved in an organic solvent, and water and electrolyte.

12. Method according to any one of Claims 1 to 9, **characterized in that** the mixture to be separated is a mixture of polyether polyol and water and electrolyte.

13. Method according to Claim 2 or 4, **characterized in that** the hydrophobic coating has been produced by the step of applying a smooth hydrophobic coating to a previously roughened surface of separating means.

14. Method according to Claim 2 or 4, **characterized in that** the hydrophobic coating has been produced by the step of applying a rough hydrophobic coating to a surface of separating means.

## Revendications

1. Procédé de séparation d'une phase organique d'un mélange d'une solution organique et d'eau contenant un électrolyte, **caractérisé en ce que** le mélange à séparer est acheminé sur un agent de séparation hydrophobe poreux, notamment une membrane organophile ou une plaque perforée ou une structure textile plate en fil métallique, ou notamment un tissu, un tricot, un feutre muni d'un revêtement hydrophobe, une partie de la phase organique du mélange traverse l'agent de séparation, tandis que la phase aqueuse contenant un électrolyte est retenue en totalité ou en une grande partie conjointement avec le reste de la phase organique.

2. Procédé selon la revendication 1, **caractérisé en ce que** l'agent de séparation est une membrane organophile et est constitué de céramique, métal, polymère, verre ou de matériaux composites appropriés en céramique et verre ou en polymère et céramique ou en métal, et comprend un revêtement avec un matériau hydrophobe.

3. Procédé selon les revendications 1 à 2, **caractérisé en ce que** la membrane hydrophile est constituée de céramique, métal, polymère, verre ou de matériaux composites appropriés en céramique et verre ou en polymère et céramique ou en métal, et est hydrophobe sans revêtement.

4. Procédé selon la revendication 2 ou 3, **caractérisé en ce que** la membrane organophile est une membrane céramique hydrophobée, notamment à base d'Al₂O₃, TiO₂, ZrO₂ ou SiC.

5. Procédé selon la revendication 2 ou 3, **caractérisé en ce que** la membrane organophile est constituée d'un polymère organophile, notamment choisi parmi : le polypropylène, le polytétrafluoroéthylène ou le polyfluorure de vinylidène.

6. Procédé selon la revendication 4 ou 5, **caractérisé en ce que** la membrane organophile présente une largeur de pores de 0,05 µm à 10 µm, de préférence de 0,1 µm à 5 µm.

7. Procédé selon la revendication 1, **caractérisé en ce qu'**en tant qu'agent de séparation hydrophobe poreux, un treillis métallique tissé muni d'un revêtement hydrophobe est utilisé, et comprend un revêtement avec un matériau hydrophobe et/ou organophile, choisi dans une liste comprenant les matériaux suivants, sans toutefois y être limitée : Teflon® AF, une dispersion de PTFE, les isocyanates, les polymères organiques et/ou fluorés ou le latex, les copolymères hybrides, notamment les copolymères diséquencés constitués par une séquence polysilsesquioxane, de préférence poly(méthylsilsesquioxane) ou poly(stéarylsilsesquioxane), et une séquence polymère hydrophobe choisie dans la série constituée par le polystyrène, le polyacrylate de méthyle, le polyacrylate d'éthylhexyle, le polyméthacrylate de méthyle, le polyméthacrylate de décyle, les acides gras, les silanes, les vernis, les silicones, les huiles de silicone ou les polysilsesquioxanes, ou les dépôts organiques et/ou fluorés, appliqués par un procédé plasma, ou un mélange de différents agents de revêtement.

8. Procédé selon la revendication 7, **caractérisé en ce qu'**un tamis hydrophobe sans modification supplémentaire de la surface est utilisé en tant qu'agent de séparation hydrophobe poreux.

9. Procédé selon la revendication 7 ou 8, **caractérisé en ce que** le treillis métallique présente une largeur de mailles < 500 µm, de préférence < 5 µm.

10. Procédé selon l'une quelconque des revendications 1 à 9, **caractérisé en ce que** le mélange à séparer est une dispersion de type émulsion d'un solvant ou d'un polymère organique aromatique ou aliphatique non miscible avec l'eau et d'eau et d'un électrolyte.

11. Procédé selon l'une quelconque des revendications 1 à 9, **caractérisé en ce que** le mélange à séparer est une dispersion de type émulsion de polycarbonate dissous dans un solvant organique et d'eau et d'un électrolyte.

12. Procédé selon l'une quelconque des revendications 1 à 9, **caractérisé en ce que** le mélange à séparer est un mélange de polyéther-polyol et d'eau et d'un électrolyte.

13. Procédé selon la revendication 2 ou 4, **caractérisé en ce que** le revêtement hydrophobe est réalisé par application d'un revêtement hydrophobe lisse sur une surface de l'agent de séparation au préalable rugosifiée.

14. Procédé selon la revendication 2 ou 4, **caractérisé en ce que** le revêtement hydrophobe est réalisé par application d'un revêtement hydrophobe rugueux sur une surface de l'agent de séparation.
